# EUROPEAN PATENT APPLICATION

(11) **EP 4 737 581 A1**
(43) Date of publication of application: **06.05.2026**
(21) Application number: 24210045.1
(22) Date of filing: 31.10.2024
(51) Int. Cl.: C12P 13/00

(54) **NOVEL SPERMIDINE AND AGMATINE PRODUCING BACILLUS STRAINS**

(71) Applicant: Evonik Operations GmbH, 45128 Essen (DE)
(72) Inventor: SPECKMANN, Bodo, 63796 Kahl (DE); BREMGES, Andreas, 33613 Bielefeld (DE); TOM DIECK, Heike, 61381 Friedrichsdorf (DE); EHRING, Ellen, 48308 Senden (DE); OCHROMBEL, Ines, 50127 Bergheim (DE); KLEINBÖLTING, Jessica, 33619 Bielefeld (DE); WIEGRÄBE, Iris, 33613 Bielefeld (DE); VOGEL, Marie-Luise, 33659 Bielefeld (DE)
(74) Representative: Evonik Patent Association

(57) **Abstract**

The present invention concerns novel spermidine and agmatine producing bacterial strains belonging to the species *Bacillus subtilis, Bacillus pumilus* and *Bacillus altitudinis* and preparations thereof as well as food, feed, pharmaceutical, cosmetical, agricultural and laundry compositions containing such strains or preparations thereof and concerns further generally the use of strains of the species *Bacillus pumilus* and *Bacillus altitudinis* to produce spermidine or agmatine, in particular in food, feed, pharmaceutical, cosmetical, agricultural, cleaning and laundry compositions.

## Description

The present invention concerns novel spermidine and agmatine producing bacterial strains belonging to the species *Bacillus subtilis, Bacillus pumilus* and *Bacillus altitudinis* and preparations thereof as well as food, feed, pharmaceutical, cosmetical, agricultural and laundry compositions containing such strains or preparations thereof and concerns further generally the use of strains of the species *Bacillus pumilus* and *Bacillus altitudinis* to produce spermidine or agmatine, in particular in food, feed, pharmaceutical, cosmetical, agricultural, cleaning and laundry compositions.

Spermidine (spd) is a biologically active polyamine with beneficial functions for animals, humans, and plants (Madeo, Eisenberg et al. 2018). It mediates and mimics the effects of (intermittent) fasting via e.g., the induction of autophagy. The induction of spermidine levels during fasting has recently been described to be indispensable for triggering autophagy and extension of animals' life span (PMID 39117797, 39212197). In addition to supporting human health, spermidine has also been ascertained to promote growth of plants and aid in detergent formulations (Xie, Wu et al. 2014)

Human applications of spermidine include its use as food ingredient or dietary supplement to promote longevity, cardio- and neuroprotection, as well as its use in skincare and hair care products. Dietary sources of spermidine include e.g. wheat germs, soy, seeds, and mushrooms. The usefulness of these sources is limited by the fact that they contain only small amounts of spermidine and by the presence of dietary restrictions, food intolerances, practice of exclusion diets or simply by their limited availability in normal diets.

In order to achieve a repeated or continuous supply of spermidine from these dietary sources their regular application is mandatory, which further limits their usability.

Beyond nutrition, the gut microbiota is a relevant source of spermidine, and finding bacterial taxa that produce it is a promising approach to make it available for the applications mentioned above. While few taxa *-Bacteroides, Fusobacterium, Bacillus subtilis* OKB105(Xie, Wu et al. 2014), *Bacillus amyloliquefaciens* SQR9, and *Heyndrickxia coagulans* YF1, *Heyndrickxia-coagulans* DSM 1, and *Bifidobacterium infantis-* have been identified or claimed to produce spermidine (Chen, Liu et al. 2017, Zou, Zhao et al. 2022, Lee, Kwon et al. 2023, Suzuki, Fujiwara et al. 2023)the state-of-the-art demonstrates a lack of robust, defined bacterial strains that produce and secrete high levels of spermidine in the context of relevant technical and/or physiological conditions, substrates, and matrices. CN 112111536 discloses the genetic engineering and use of recombinant bacteria to synthesize spermidine. Similarly, *Escherichia coli* and *Bacillus amyloquifaciens* strains have been bioengineered to produce spermidine(Caffaratti, Plazy et al. 2022, Zou, Zhao et al. 2022). Spermidine production by non-GMO microbial strains has been reported, but levels of spermidine found in supernatants of cultured strains do not exceed 5-6 mg/I(Lee, Kwon et al. 2023). Therefore, there is a need to provide new microbial strains and/or species that can produce high yields of spermidine under practically relevant conditions and from available sources.

By applying bioinformatic and cell culture techniques, novel spermidine producing strains of the species *Bacillus subtilis, Bacillus pumilus* and *Bacillus altitudinis* could be identified which produce high amounts of spermidine, in particular higher amounts than the benchmark strain *Heyndrickxia coagulans* DSM 1. While for strains of the species *Bacillus subtilis* it was known before that they are able to produce spermidine, it was unknown and surprising that strains of the species *Bacillus pumilus* and *Bacillus altitudinis* are also able to produce spermidine in significant amounts. This was the more surprising, because the strains which could be identified as effective spermidine producers according to the invention do not possess the genes encoding for the expected spermidine transporter proteins PotA, PotB, PotC and PotD, which were expected to be necessary for an effective transport and thus accumulation of spermidine into the surrounding medium.

Furthermore, it turned out that the identified strains are besides spermidine also producing agmatine as a beneficial co-product in relatively high amounts.

The subject matter of the invention has some significant advantages compared to the state of the art: the microorganisms of the invention can be applied in the form of dietary supplements or functional foods, optionally combined with L-arginine or sources thereof such as proteins or protein hydrolysates in a form that is suitable for any type of allergen requirements, exclusion diets or particular nutritional needs, to provide the health benefits of spermidine via its sustained production in the gastrointestinal tract. As the microorganisms of the invention can engraft in the gastrointestinal tract and L-arginine and sources thereof are components of the normal diet, the single or occasional application of the microorganisms of the invention may be sufficient to provide the host with a continuous supply of spermidine via its production *in vivo* from substrates contained in the diet or released upon gut microbial metabolism.

The microorganisms may be applied in the form of (endo)spores, allowing for their use under harsh manufacturing, storage, and intestinal conditions. Similarly, the strains can be used for the production of spermidine-rich ferments, to be included in functional foods, dietary supplements, skincare or haircare products. The fact that *Bacillus pumilus* strains have strong gluten-degrading capacity enables the production of gluten-free spermidine-rich extracts from wheat germs. The microorganisms of the invention and preparations thereof can also be used for seed treatments, as soil fertilizers or added to laundry formulations.

Thus, a first subject of the present invention are microorganisms, as identified as effective spermidine and/or agmatine producers according to the invention, and microbial preparations thereof, selected from the following group:
a) *Bacillus subtilis* DSM 35086;
b) *Bacillus subtilis* DSM 35087;
c) *Bacillus subtilis* DSM 35085;
d) *Bacillus subtilis* DSM 33561;
e) *Bacillus pumilus* DSM 35088;
f) *Bacillus pumilus* DSM 35089;
g) *Bacillus pumilus* DSM 35090;
h) *Bacillus pumilus* DSM 35091 ;
i) *Bacillus pumilus* DSM 35093;
j) *Bacillus altitudinis* DSM 35092;
k) A mutant of any of the strains according to (a) to (j) with a sequence identity of at least 95 %, preferably of at least 97, 98 or 99 %, more preferably of at least 99.5, 99.8 or 99.9 %, in particular of at least 99.95, 99.98 or 99.99 %, to the genomic sequence of any of the strains according to (a) to (j);
l) A preparation of a microorganism according to (a) to (k), wherein the preparation is preferably an optionally concentrated or dried fermentation broth, supernatant of a fermentation broth, ferment, cell lysate or cell extract of a microorganism according to (a) to (k), wherein the preparation may be a cell-free preparation or may contain living or dead cells or cell debris of such a microorganism or any combination thereof.

The microorganisms and microbial preparations according to the invention are able enhance human, animal, and plant health by their exceptional ability to produce spermidine and making it available to the body or to a plant either upon formation *in vivo* or upon administration of *in vitro* produced spermidine.

Due to the known effects of spermidine, the microorganisms and microbial preparations of the invention can be used in different ways, in particular in food, feed, and agricultural compositions, in particular in functional foods, pet foods, nutraceuticals and foods for special medical purposes (FSMP), in food and feed additives, in food and feed supplements, in pharmaceutical compositions, in cosmetical compositions, in particular for topical use, as well as in personal care products, in fertilizers, in soil amendments, and in plant growth regulators, which all are accordingly also subject matter of the invention.

The application of the microorganisms and microbial preparations of the invention can accordingly occur in particular by oral application or topical or agricultural application.

Thus, a further subject of the invention is also a composition, in particular as mentioned before, containing a microorganism or microbial preparation according to the invention, in particular at least one microorganism or microbial preparation according to the invention, preferably selected from the following group:
a) *Bacillus subtilis* DSM 35086;
b) *Bacillus subtilis* DSM 35087;
c) *Bacillus subtilis* DSM 35085;
d) *Bacillus subtilis* DSM 33561;
e) *Bacillus pumilus* DSM 35088;
f) *Bacillus pumilus* DSM 35089;
g) *Bacillus pumilus* DSM 35090;
h) *Bacillus pumilus* DSM 35091 ;
i) *Bacillus pumilus* DSM 35093;
j) *Bacillus altitudinis* DSM 35092;
k) A mutant of any of the strains according to (a) to (j) with a sequence identity of at least 95 %, preferably of at least 97, 98 or 99 %, more preferably of at least 99.5, 99.8 or 99.9 %, in particular of at least 99.95, 99.98 or 99.99 %, to the genomic sequence of any of the strains according to (a) to (j);
l) A preparation of a microorganism according to (a) to (k), wherein the preparation is preferably an optionally concentrated or dried fermentation broth, supernatant of a fermentation broth, ferment, cell lysate or cell extract of a microorganism according to (a) to (k), wherein the preparation may be a cell-free preparation or may contain living or dead cells or cell debris of such a microorganism or any combination thereof.

Thus, a further subject of the invention in in particular also a pharmaceutical composition, containing a microorganism and/or microbial preparation according to the invention and at least one pharmaceutically acceptable carrier.

A further subject of the invention is accordingly in particular also a composition, in particular a pharmaceutical composition, for use in the treatment, prevention or mitigation of a disease selected from dementia, Alzheimer's disease, Parkinson's disease, arthritis, hypertension, arterial stiffness, heart failure, myopathy, sepsis or cardiovascular diseases and/or for use in increasing the life span, promoting longevity, improving male or female fertility, promoting hair or nail growth, improving immunogenicity after vaccination, improving mitochondrial respiration.

A further subject of the invention is therefore also the use of a microorganism or microbial preparation or of a composition of the invention in the manufacture of a pharmaceutical composition, in particular for treating, preventing or mitigating the course of a disease selected from dementia, Alzheimer's disease, Parkinson's disease, arthritis, hypertension, arterial stiffness, heart failure, myopathy, sepsis or cardiovascular diseases and/or for increasing the life span, promoting longevity, improving male or female fertility, promoting hair or nail growth, improving immunogenicity after vaccination, improving mitochondrial respiration.

A further subject of the invention is therefore also the non-medical use of a microorganism or microbial preparation or of a composition or of a product of the invention for improving skin firmness, elasticity, viscoelasticity, resilience or hydration, for reducing or preventing wrinkles or skin irritation or for promoting hair and nail growth.

Thus, a further subject of the invention is also a product, in particular a dietary supplement, an agricultural product or a personal care article, containing a microorganism or microbial preparation according to the invention, in particular at least one microorganism or microbial preparation according to the invention, preferably selected from the following group:
a) *Bacillus subtilis* DSM 35086;
b) *Bacillus subtilis* DSM 35087;
c) *Bacillus subtilis* DSM 35085;
d) *Bacillus subtilis* DSM 33561;
e) *Bacillus pumilus* DSM 35088;
f) *Bacillus pumilus* DSM 35089;
g) *Bacillus pumilus* DSM 35090;
h) *Bacillus pumilus* DSM 35091;
i) *Bacillus pumilus* DSM 35093;
j) *Bacillus altitudinis* DSM 35092;
k) A mutant of any of the strains according to (a) to (j) with a sequence identity of at least 95 %, preferably of at least 97, 98 or 99 %, more preferably of at least 99.5, 99.8 or 99.9 %, in particular of at least 99.95, 99.98 or 99.99 %, to the genomic sequence of any of the strains according to (a) to (j);
l) A preparation of a microorganism according to (a) to (k), wherein the preparation is preferably an optionally concentrated or dried fermentation broth, supernatant of a fermentation broth, ferment, cell lysate or cell extract of a microorganism according to (a) to (k), wherein the preparation may be a cell-free preparation or may contain living or dead cells or cell debris of such a microorganism or any combination thereof.

All deposited strains of the invention were identified by screening of naturally occurring isolates and were deposited at the DSMZ (Leibniz-lnstitute DSMZ-German Collection of Microorganisms and Cell Cultures, Inhoffenstraße 7B, 38124 Braunschweig, Germany) under the provisions of the Budapest Treaty on the International Recognition of the Deposit of Microorganisms for the Purpose of Patent Procedure in the name of the applicant Evonik Operations GmbH. While the strain *Bacillus subtilis* DSM 33561 was deposited on June 25, 2020, all other strains, i.e. the strains *Bacillus subtilis* DSM 35085, DSM 35086 and DSM 35087, the strains *Bacillus pumilus* DSM 35088, DSM 35089, DSM 35090, DSM 35091 and DSM 35093, as well as the strain *Bacillus altitudinis* DSM 35092 were deposited on July 5, 2024.

The *Bacillus subtilis* DSM 35086 exhibits the following characterizing sequences: a 16S rDNA sequence according to SEQ ID NO: 1, an groL sequence according to SEQ ID NO: 2, a gyrB sequence according to SEQ ID NO: 3, an rpoB sequence according to SEQ ID NO: 4, an yqfD sequence according to SEQ ID NO: 5 and a sequence according to SEQ ID NO: 6 which encodes an orphan membrane protein.

Accordingly, the mutants of the strain DSM 35086 preferably exhibit a 16S rDNA sequence, an groL sequence, a gyrB sequence, a rpoB sequence, an yqfD sequence and/or said sequence encoding an orphan membrane protein, which are at least 99 or 99.5 %, more preferably at least 99.8 or 99.9 %, above all 100 % identical to the respective sequences of the strain DSM 35086.

The mutants of the strain DSM 35086 have preferably further a sequence identity of at least 99 %, more preferably of at least 99.5 %, in particular of at least 99.8 or 99.9 % to the DNA, in particular to the genomic DNA, of the strain DSM 35086.

The *Bacillus subtilis* DSM 35087 exhibits the following characterizing sequences: a 16S rDNA sequence according to SEQ ID NO: 7, an groL sequence according to SEQ ID NO: 8, a gyrB sequence according to SEQ ID NO: 9, an rpoB sequence according to SEQ ID NO: 10 and an yqfD sequence according to SEQ ID NO: 11.

Accordingly, the mutants of the strain DSM 35087 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35087.

The mutants of the strain DSM 35087 have preferably further a sequence identity of at least 99 %, more preferably of at least 99.5 %, in particular of at least 99.8 or 99.9 % to the DNA, in particular the genomic DNA, of the strain DSM 35087.

The *Bacillus subtilis* DSM 35085 exhibits the following characterizing sequences: a 16S rDNA sequence according to SEQ ID NO: 12, an groL sequence according to SEQ ID NO: 13, a gyrB sequence according to SEQ ID NO: 14, an rpoB sequence according to SEQ ID NO: 15, an yqfD sequence according to SEQ ID NO: 16 and a sequence according to SEQ ID NO: 17 which encodes peptidase G2.

Accordingly, the mutants of the strain DSM 35085 preferably exhibit a 16S rDNA sequence, an groL sequence, a gyrB sequence, a rpoB sequence, an yqfD sequence and/or said sequence encoding peptidase G2, which are at least 99 or 99.5 %, more preferably at least 99.8 or 99.9 %, above all 100 % identical to the respective sequences of the strain DSM 35085.

The mutants of the strain DSM 35085 have preferably further a sequence identity of at least 99 %, more preferably of at least 99.5 %, in particular of at least 99.8 or 99.9 % to the DNA, in particular to the genomic DNA, of the strain DSM 35085.

The *Bacillus pumilus* DSM 35088 exhibits the following characterizing sequences: a 16S rDNA sequence according to SEQ ID NO: 18, an groL sequence according to SEQ ID NO: 19, a gyrB sequence according to SEQ ID NO: 20, an rpoB sequence according to SEQ ID NO: 21 and an yqfD sequence according to SEQ ID NO: 22.

Accordingly, the mutants of the strain DSM 35088 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35088.

The mutants of the strain DSM 35088 have preferably further a sequence identity of at least 99 %, more preferably of at least 99.5 %, in particular of at least 99.8 or 99.9 % to the DNA, in particular the genomic DNA, of the strain DSM 35088.

The *Bacillus pumilus* DSM 35089 exhibits the following characterizing sequences: a 16S rDNA sequence according to SEQ ID NO: 23, an groL sequence according to SEQ ID NO: 24, a gyrB sequence according to SEQ ID NO: 25, an rpoB sequence according to SEQ ID NO: 26 and an yqfD sequence according to SEQ ID NO: 27.

Accordingly, the mutants of the strain DSM 35089 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35089.

The mutants of the strain DSM 35089 have preferably further a sequence identity of at least 99 %, more preferably of at least 99.5 %, in particular of at least 99.8 or 99.9 % to the DNA, in particular the genomic DNA, of the strain DSM 35089.

The *Bacillus pumilus* DSM 35090 exhibits the following characterizing sequences: a 16S rDNA sequence according to SEQ ID NO: 28, an groL sequence according to SEQ ID NO: 29, a gyrB sequence according to SEQ ID NO: 30, an rpoB sequence according to SEQ ID NO: 31 and an yqfD sequence according to SEQ ID NO: 32.

Accordingly, the mutants of the strain DSM 35090 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35090.

The mutants of the strain DSM 35090 have preferably further a sequence identity of at least 99 %, more preferably of at least 99.5 %, in particular of at least 99.8 or 99.9 % to the DNA, in particular the genomic DNA, of the strain DSM 35090.

The *Bacillus pumilus* DSM 35091 exhibits the following characterizing sequences: a 16S rDNA sequence according to SEQ ID NO: 33, an groL sequence according to SEQ ID NO: 34, a gyrB sequence according to SEQ ID NO: 35, an rpoB sequence according to SEQ ID NO: 36 and an yqfD sequence according to SEQ ID NO: 37.

Accordingly, the mutants of the strain DSM 35091 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35091.

The mutants of the strain DSM 35091 have preferably further a sequence identity of at least 99 %, more preferably of at least 99.5 %, in particular of at least 99.8 or 99.9 % to the DNA, in particular the genomic DNA, of the strain DSM 35091.

The *Bacillus pumilus* DSM 35093 exhibits the following characterizing sequences: a 16S rDNA sequence according to SEQ ID NO: 38, an groL sequence according to SEQ ID NO: 39, a gyrB sequence according to SEQ ID NO: 40, an rpoB sequence according to SEQ ID NO: 41 and an yqfD sequence according to SEQ ID NO: 42.

Accordingly, the mutants of the strain DSM 35093 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35093.

The mutants of the strain DSM 35093 have preferably further a sequence identity of at least 99 %, more preferably of at least 99.5 %, in particular of at least 99.8 or 99.9 % to the DNA, in particular the genomic DNA, of the strain DSM 35093.

The *Bacillus altitudinis* DSM 35092 exhibits the following characterizing sequences: a 16S rDNA sequence according to SEQ ID NO: 43, an groL sequence according to SEQ ID NO: 44, a gyrB sequence according to SEQ ID NO: 45, an rpoB sequence according to SEQ ID NO: 46 and an yqfD sequence according to SEQ ID NO: 47.

Accordingly, the mutants of the strain DSM 35092 preferably exhibit a 16S rDNA sequence, an groL sequence, a gyrB sequence, a rpoB sequence and an yqfD sequence which are at least 99 or 99.5 %, more preferably at least 99.8 or 99.9 %, above all 100 % identical to the respective sequences of the strain DSM 35092.

The mutants of the strain DSM 35092 have preferably further a sequence identity of at least 99 %, more preferably of at least 99.5 %, in particular of at least 99.8 or 99.9 % to the DNA, in particular the genomic DNA, of the strain DSM 35092.

The *Bacillus subtilis* DSM 33561 exhibits the following characterizing sequences: a 16S rDNA sequence according to SEQ ID NO: 48, a groL sequence according to SEQ ID NO: 49, a gyrB sequence according to SEQ ID NO: 50, an rpoB sequence according to SEQ ID NO: 51 and an yqfD sequence according to SEQ ID NO: 52.

Accordingly, the mutants of the strain DSM 33561 preferably exhibit a 16S rDNA sequence, a groL sequence, a gyrB sequence, a rpoB sequence and an yqfD sequence, which are at least 99 or 99.5 %, more preferably at least 99.8 or 99.9 %, above all 100 % identical to the respective sequences of the strain DSM 33561.

The mutants of the strain DSM 33561 have preferably further a sequence identity of at least 99 %, more preferably of at least 99.5 %, in particular of at least 99.8 or 99.9 % to the DNA, in particular to the genomic DNA, of the strain DSM 33561.

The mutants of the invention can principally be obtained by any kind of method, i.e., by GMO- or non-GMO methods, but preferably the mutants are not genetically modified, i.e., non-GMO. This means that the mutants of the deposited strains are preferably either also naturally occurring microorganisms or spontaneous mutants of such naturally occurring microorganisms, in particular of the deposited strains, or microorganisms which are obtained by another method which is classified as non-GMO. The term "spontaneous mutant" refers to mutants that arise from naturally occurring microorganisms and/or parent strains without genetically modifying the microorganisms by applying classical gene technological and/or biotechnological methods like site-directed mutagenesis. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible.

Spontaneous mutants might further, but less preferably, be obtained by using mutagens, i.e., chemical substances which induce the formation of mutants. As formation of spontaneous mutants by using mutagens is less preferred, in a preferred embodiment of the invention the spontaneous mutants and/or non-GMO mutants are obtained without the use of such mutagens. If the mutants are obtained by applying gene technological and/or biotechnological methods like site-directed mutagenesis, then preferably methods are applied which are classified as non-GMO. A non-GMO method according to the invention is preferably characterized in that the method does not involve introduction of heterologous genetic information into the microorganism. In a particularly preferred embodiment of the invention the microorganisms of the invention are naturally non-occurring mutants, in particular non-GMO and/or spontaneous mutants as defined before.

The mutants of the invention have preferably the same or very similar characteristics like the parent strain from which they are derived. The mutants of the invention are in particular preferably able to produce at least 90 % of the amount of spermidine and/or agmatine in comparison to the amount of spermidine and/or agmatine which is produced by the parent strain from which they are derived, if they are cultivated under the same conditions like the parent strain, in particular if they are cultivated in LBG medium (Luria Bertani medium, supplemented with 1 g/L glucose), in particular if cultivated for 24 hours. Preferably the mutants are able to produce at least the same amount of spermidine and/or agmatine in comparison to the parent strain from which they are derived.

The strains and mutants of the invention are further preferably able to produce more spermidine and/or agmatine than the benchmark strain *Heyndrickxia coagulans,* in particular if cultivated under the same conditions. A suitable medium for the comparison of the ability to produce spermidine and/or agmatine is Luria Bertani medium supplemented with 1 g/L glucose (also called LBG medium) and further supplemented with 0.5 g/L L-arginine and 0.5 g/L L-methionine. The LBG medium supplemented with L-arginine and L-methionine as preferably used for testing and comparing the ability to produce spermidine and/or agmatine exhibits accordingly the following composition: 5 g/L yeast extract, 10 g/L peptone from casein,10g/L sodium chloride, 1g/L glucose monohydrate, 0.5 g/L L-arginine and 0.5 g/L L-methionine, Very preferably the ability of the microorganisms to produce spermidine and/or agmatine is tested as disclosed in working example 2 below.

The microorganisms of the invention are preferably able to produce at least 2.5 mg/L, preferably at least 3, 4 or 5 mg/L, more preferably at least 5, 7 or 8 mg/L, above all at least 9 or 10 mg/L, spermidine in an appropriate fermentation medium, in particular in LBG medium supplemented with L-arginine and L-methionine as disclosed before, wherein the mutants of the deposited strains are preferably able to produce at least 90 %, more preferably at least the same amount of spermidine in comparison to the parent strain, from which they are derived. The microbial preparations of the invention accordingly contain spermidine preferably in the amounts as disclosed before.

The microorganisms of the invention are further preferably able to produce at least 2 mg/L, preferably at least 5 or 10 mg/L, more preferably at least 20 or 30 mg/L, above all at least 35 or 40 mg/L, agmatine in an appropriate fermentation medium, in particular in LBG medium supplemented with L-arginine and L-methionine as disclosed before, wherein the mutants of the deposited strains are preferably able to produce at least 90 %, more preferably at least the same amount of agmatine in comparison to the parent strain, from which they are derived. The microbial preparations of the invention accordingly contain agmatine preferably in the amounts as disclosed before.

In particular, the strain DSM 35086 and mutants thereof are preferably able to produce at least 10 mg/L, preferably at least 11 mg/L, spermidine and/or at least 5 mg/L, preferably at least 6 mg/L agmatine, in particular if cultivated in LBG medium, above all if cultivated in LBG medium supplemented with L-arginine and L-methionine as disclosed before for 24 hours.

In particular, the strain DSM 35087 and mutants thereof are preferably able to produce at least 9 mg/L, preferably at least 10 mg/L, spermidine and/or at least 5 mg/L, preferably at least 6 mg/L agmatine, in particular if cultivated in LBG medium, above all if cultivated in LBG medium supplemented with L-arginine and L-methionine as disclosed before for 24 hours.

In particular, the strain DSM 35085 and mutants thereof are preferably able to produce at least 8 mg/L, preferably at least 9 mg/L, spermidine and/or at least 1 mg/L, preferably at least 2 mg/L agmatine, in particular if cultivated in LBG medium, above all if cultivated in LBG medium supplemented with L-arginine and L-methionine as disclosed before for 24 hours.

In particular, the strain DSM 33561 and mutants thereof are preferably able to produce at least 7 mg/L, preferably at least 8 mg/L, spermidine and/or at least 4 mg/L, preferably at least 5 mg/L agmatine, in particular if cultivated in LBG medium, above all if cultivated in LBG medium supplemented with L-arginine and L-methionine as disclosed before for 24 hours.

In particular, the strain DSM 35088 and mutants thereof are preferably able to produce at least 5 mg/L, preferably at least 6 mg/L, spermidine and/or at least 8 mg/L, preferably at least 9 mg/L agmatine, in particular if cultivated in LBG medium, above all if cultivated in LBG medium supplemented with L-arginine and L-methionine as disclosed before for 24 hours.

In particular, the strain DSM 35089 and mutants thereof are preferably able to produce at least 1 mg/L, preferably at least 2 mg/L, spermidine and/or at least 30 mg/L, preferably at least 35 mg/L agmatine, in particular if cultivated in LBG medium, above all if cultivated in LBG medium supplemented with L-arginine and L-methionine as disclosed before for 24 hours.

In particular, the strain DSM 35090 and mutants thereof are preferably able to produce at least 4 mg/L, preferably at least 5 mg/L, spermidine and/or at least 20 mg/L, preferably at least 25 mg/L agmatine, in particular if cultivated in LBG medium, above all if cultivated in LBG medium supplemented with L-arginine and L-methionine as disclosed before for 24 hours.

In particular, the strain DSM 35091 and mutants thereof are preferably able to produce at least 5 mg/L, preferably at least 6 mg/L, spermidine and/or at least 35 mg/L, preferably at least 40 mg/L agmatine, in particular if cultivated in LBG medium, above all if cultivated in LBG medium supplemented with L-arginine and L-methionine as disclosed before for 24 hours.

In particular, the strain DSM 35093 and mutants thereof are preferably able to produce at least 5 mg/L, preferably at least 6 mg/L, spermidine and/or at least 10 mg/L, preferably at least 13 mg/L agmatine, in particular if cultivated in LBG medium, above all if cultivated in LBG medium supplemented with L-arginine and L-methionine as disclosed before for 24 hours.

In particular, the strain DSM 35092 and mutants thereof are preferably able to produce at least 5 mg/L, preferably at least 6 mg/L, spermidine and/or at least 25 mg/L, preferably at least 29 mg/L agmatine, in particular if cultivated in LBG medium, above all if cultivated in LBG medium supplemented with L-arginine and L-methionine as disclosed before for 24 hours.

The microorganisms of the invention may be present, in particular in the compositions and products of the present invention, as spores (which are dormant), as vegetative cells (which are growing), as transition state cells (which are transitioning from growth phase to sporulation phase) or as a combination of at least two, in particular all of these types of cells. In a preferred embodiment, the compositions and products of the invention comprise mainly or only spores, wherein preferably at least 60, 70, 80 or 90 % of the microorganisms as contained in the composition are spores. But besides that the cells can also be used in non-living, inactivated form, or as a combination of living and inactivated cells, as preparations containing inactivated cells still comprise spermidine and/or agmatine, as previously produced by the cells.

Accordingly, preparations of the microorganisms of the invention, which are a preferred subject of the invention, may be preparations containing intact cells, inactivated cells, cell debris or mixtures thereof. Further, the preparations may also be cell-free preparations, wherein the cell-free preparations may contain cell debris or may be free of cell debris. Particularly preferred examples for preparations of the microorganisms of the invention are the fermentation broth, as obtained after finishing the fermentation of the cells, the supernatant of the fermentation broth, which is obtained by separating all or the major part of the cells from the fermentation broth, as well as cell lysates and cell extracts, i.e. cytosol preparations, which can be obtained by breaking the microbial cells. Such preparations may also be used in concentrated or dried form, wherein the dried form has preferably a total dry matter content of at least 90 wt.-%, more preferably of at least 95 wt.-%.

The term "fermentation broth" according to the invention refers to the product of a cultivation of bacteria in a suitable fermentation medium. Methods for producing such fermentation broths are well known to those skilled in the art. The fermentation broths of the present invention can for example be obtained by culturing the strains by using the media, conditions and methods as described in US 6,060,051, EP 0287699, US 2014/0010792 or in the FAO Report 179 (2016): "Probiotics in Animal Nutrition". Conventional large-scale microbial culture processes include submerged fermentation, solid state fermentation, or liquid surface culture. Typically, fermentation is carried out, until a certain cell density is reached like an optical density (OD 600) of about 5 to 25, more preferably 10 to 22, above all 15 to 20. The fermentation broths preferably contain cells in an amount of 1×10³ to 1×10¹³ CFU, more preferably in an amount of 1×10⁵ to 1×10¹² CFU, above all in an amount of 1×10⁷ to 1×10¹¹ CFU per ml of fermentation broth.

The term "fermentation broth" according to the invention refers to the direct product of the cultivation, i.e. a suspension preferably containing cells in the amount as mentioned in the previous paragraph, as well as to concentrated and dried forms of such a fermentation broth, wherein the dried form ("dried fermentation broth") preferably has a total dry matter content of at least 95 wt.-%.

Drying of the preparations of the invention can be carried out by applying methods as generally known to those skilled in the art, in particular by evaporation of water, freeze-drying, lyophilization, spray drying, spray granulation, fluidized bed drying, vacuum drying, tray drying, drum drying and combinations thereof. After drying, the dried preparations as obtained can be further worked up, in particular by grinding and/or granulation.

Cell-free preparations of the microorganisms of the invention can be obtained by centrifugation, filtration, in particular ultrafiltration or microfiltration, decantation and/or flotation of the fermentation broth or of a suspension as obtained after breaking the microbial cells. Depending on the technique used, these cell-free preparations may not be completely devoid of cells, but may still comprise a smaller amount of cells, in particular up to 20 wt.-% or up to 10 wt.-% of cells. As the cells produce and secret compounds like metabolites, enzymes and/or peptides into the surrounding medium, the supernatants, extracts and lysates of the cells comprises a mixture of such compounds, in particular metabolites, enzymes and/or peptides, as secreted and/or produced by the cells.

Cell lysates can be prepared either directly by breaking the cells of an optionally dried fermentation broth or by breaking the cells after first separating the cells from the fermentation broth mechanically, in particular by filtration, centrifugation and/or decantation.

Breaking of the cells can be carried out by applying techniques as known to those of skill in the art, in particular mechanically, chemically and/or enzymatically. Depending on the degree of force applied, a composition comprising only ruptured cells or a composition comprising a mixture of cell debris and intact cells is obtained. Homogenization of the cells may be realized for example by applying high pressure, high pH, osmotic shock, heat shock, sonication, homogenization or shearing, in particular by utilizing means selected from French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer.

After breaking of the cells, the cell debris and remaining cells, if any, can optionally be separated from the cytosol preparation thus obtained to obtain a cell extract, i.e. a cytosol preparation, which is free of cells and cell debris.

The fermentation broths of the invention, from which the cells have been removed, i.e. the cell-free supernatant and metabolites containing fractions of the fermentation broth, are also denoted as "postbiotics" or "biogenics".

A further specific preparation of the invention is a preparation containing inactivated cells, i.e. cells which are not able to grow anymore. Preparations containing inactivated cells are also known as "paraprobiotics". Such preparations can be obtained for example by heat and/or pH inactivation of the cells, preferably by heat and/or pH inactivation of a fermentation broth containing the cells.

Inactivation of the cells can be carried out, e.g., by heat treatment and/or by adjusting an acid or an alkaline pH. Heat inactivation of the cells is preferably carried out by increasing the temperature to between 60°C and 80°C and incubation for at least 30 minutes. pH inactivation at acidic pH is preferably carried out by lowering the pH to at least 3 by addition of an acid like 5M H₂SO₄ and incubation for at least 30 minutes, preferably at least one hour. pH inactivation at alkaline pH is preferably carried out by increasing the pH to at least 10 and incubation for at least 30 minutes, preferably at least one hour. Alternatively, inactivation of the cells may also be carried out by applying gamma- or UV-irradiation. In one specific embodiment of the invention a fermentation broth is used, in which at least 90 %, more preferably at least 95 or 99 %, in particular all bacterial cells are inactivated. Correspondingly, in this embodiment of the invention a fermentation broth is used which preferably contains no viable cells, at all. Such a product, which contains mainly or exclusively inactivated cells, is preferably used in cosmetical and/or topical applications and is called "ferment" in the context of the present invention.

The microbial preparations of the invention may contain besides spermidine and agmatine metabolites, enzymes and/or peptides as produced and/or secreted by the microorganisms of the invention. They may also comprise organic acids, in particular lactate. Separation of spermidine from cell debris, if necessary, can simply be carried out by centrifugation, decantation or filtration.

Preferably according to the invention always an effective amount of the microorganisms, microbial preparations, in particular fermentation broths, compositions and/or products of the invention is used in the embodiments of the invention.

Compositions and products of the invention preferably contain the microorganisms of the invention in an amount of from 1×10² to 1×10¹² CFU (colony forming units), more preferably in an amount of 1×10³ to 1×10¹⁰ CFU, above all in an amount of 1×10⁴ to 1×10⁹ CFU and/or in an amount of from 0.1 to 20 wt.-%, preferably 0.2 to 15 wt.-%, in particular 0.5 to 10 wt.-%, above all 1 to 5 wt.-%, wherein preferably the microorganisms are exclusively or mainly present as endospores.

In context with food and feed compositions of the invention, the term "effective amount" refers to an amount which effects at least one beneficial effect to the gastrointestinal tract of human beings or animals in comparison to the gastrointestinal tract of human beings or animals that have not been exposed to the microorganisms, microbial preparations and/or compositions of the invention, but may have been exposed to the same treatment and/or to the same food or feed compositions depleted by such microorganisms or microbial preparations.

In context with cosmetical compositions of the invention, the term "effective amount" refers to an amount which effects at least one beneficial effect to the human skin in comparison to a human skin that has not been exposed to the microorganisms, microbial preparations and/or compositions of the invention, but may have been exposed to the same treatment and/or to the same cosmetical composition depleted by microorganisms or microbial preparations.

The microorganisms and microbial preparations of the invention can also be provided in combination with a suitable carrier, wherein the carrier is preferably an inert formulation ingredient added to improve recovery, efficacy, or physical properties and/or to aid in packaging or administration of the microorganisms or microbial preparations. Such carriers may be used individually or in combination and can be added either as part of the fermentation medium, in the course of the fermentation or after the fermentation of the microorganisms has been ended.

The carrier is preferably selected from anti-caking agents, antioxidants, bulking agents, binders, structurants, coatings and/or protectants. Examples of useful carriers include polysaccharides (in particular starches, maltodextrins, celluloses, methylcelluloses, gums like guar gum, xanthan gum and gum arabic, wheat middlings, corn cob meal, chitosan and/or inulins), protein sources (in particular skim milk powder, sweet-whey powder, gelatine and/or soy flour), protein hydrolysates (in particular gelatine, yeast extract and/or peptones like soy peptone), peptides, sugars (in particular lactose, trehalose, sucrose, dextrose and/or maltose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), silicates (in particular clays, zeolites, Fuller's earth, clintpolite, montmorillonite, perlite, vermiculite, diatomaceous earth, talc, bentonites, kaolin clay, silica in particular precipitated silica, hydrophobic silica and/or hydrophilic silica, and/or silicate salts like aluminium, magnesium and/or calcium silicate), silica gel, silica dioxide, activated carbon, lignite, magnesium and calicum oxide.

Compositions of the invention preferably contain a substrate that can be biochemically converted to spermidine or that is required for the biosynthesis of spermidine, e.g. by serving as a cofactor of an enzyme involved in the biosynthesis of spermidine. The substrate is selected from the amino acids L-arginine, L-ornithine, L-methionine, agmatine, or putrescine or sources thereof. Preferably, the substrate is L-arginine or a salt thereof or at least one source of L-arginine or of a salt thereof (referred to as "L-arginine source"). The L-arginine source is preferably selected from peptides, protein hydrolysates, plant extracts, in particular extracts of wheat germs or soy, spirulina powder, or bacterial extracts. Instead of a direct source of L-arginine, also a source can be used, which can easily be converted enzymatically either by the microorganism of the invention or by the human or animal body into L-arginine. Sources for L-arginine are in particular also derivatives of L-arginine like L-arginine alpha-ketoglutarate or L-arginine ethylester.

Compositions of the invention, in particular as mentioned before, may also contain at least one further additive selected from carriers, in particular as mentioned before, further probiotics, prebiotics, enzymes, vitamins, minerals, further amino acids, peptides, plant extracts and algal extracts.

The at least one further probiotic microorganism is preferably contained in the compositions of the invention, if present, in an amount of about 1×10² to about 1×10¹⁰ CFU/g, more preferably in an amount of about 1×10⁴ to about 1×10⁹ CFU/g, in particular in an amount of 1×10⁶ to 1×10⁸ CFU/g. It is used preferably in form of an optionally dried fermentation broth.

Such further probiotic microorganisms are preferably bacteria selected from the genera Bacillus, Lactobacillus, Lacticaseibacillus, Lactiplantibacillus, Levilactobacillus, Bifidobacterium, Enterococcus or Pediococcus. Probiotic microorganisms of the genus Bacillus are preferably selected from the species *Bacillus subtilis, Bacillus licheniformis, Bacillus paralicheniformis, Bacillus lentus, Bacillus pumilus, Bacillus laterosporus, Bacillus coagulans, Bacillus alevi, Bacillus cereus, Bacillus badius, Bacillus thurigiensis, Bacillus amyloliquefaciens, Bacillus velezensis, Bacillus nakamurai* and *Bacillus siamensis.* Probiotic bacteria of the genus Enterococcus are preferably of the species *Enterococcus faecium.* Probiotic bacteria of the genus Pediococcus are preferably of the species *Pediococcus acidilactici.* Further suitable probiotic microorganisms are *E*. *coli* Nissle and *Clostridium butyricum.*

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used according to the invention and which may aid in the digestion of feed, are preferably selected from phytases (EC 3.1 .3.8 or 3.1 .3.26), xylanases (EC 3.2.1 .8), galactanases (EC 3.2.1 .89), galactosidases, in particular alpha-galactosidases (EC 3.2.1.22) and beta-galactosidases, proteases (EC 3.4), lipases, phospholipases, in particular phospholipases A1 (EC 3.1 .1 .32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ), lysozymes (EC 3.2.1 .17), glucanases, in particular beta- glucanases (EC 3.2.1 .4 or EC 3.2.1 .6), glucoamylases, cellulases, pectinases, glutamate decarboxylases, or any mixture thereof.

Vitamins which may be used according to the invention are for example vitamin A, vitamin D3, vitamin E, vitamin K, e.g., vitamin K3, vitamin B12, biotin, choline, vitamin B1, vitamin B2, vitamin B6, niacin, folic acid and pantothenate, e.g. Ca-D-pantothenate, or combinations thereof.

Minerals and trace elements which may be used according to the invention are for example boron, cobalt, lithium, chloride, chromium, copper, fluoride, iodine, iron, manganese, molybdenum, selenium, zinc, calcium, phosphorous, magnesium, potassium, or sodium, or combinations thereof. Amino acids which may be used according to the invention are for example lysine, alanine, threonine, methionine or tryptophan, or combinations thereof.

Further amino acids which may be used according to the invention are in particular proteinogenic amino acids as known to those skilled in the art, in particular L-threonine, L-methionine, L-valine, L-lysine, L-glycine, and L-tryptophan.

Dipeptides which may be used according to the invention are in particular any dipeptides of proteinogenic amino acids.

Plant extracts are according to the invention preferably selected from extracts from broccoli, olive fruit, pomegranate, blackcurrant, blueberry, bilberry, sea buckthorn, camu camu, boysenberry, curcuma, ginger, garlic, grape seeds, acai berry, aronia, goji berry, horseradish, rice, beans, kudzu, bamboo, hokkaido, boswellia serrata, spirulina, panax ginseng, cannabidiol, rose hip, pu erh, sea grass, sencha, echinacea, rhodiola rosea, and green tea leaves.

Compositions of the invention, in particular feed, food and pharmaceutical compositions of the invention, preferably comprise at least one further feed or food additive.

Suitable typical feed or food additives which may be contained in the feed, food and pharmaceutical compositions according to the invention include one or more of the following: proteins, carbohydrates, fats, feed concentrates, silage, mashfeed, probiotics, prebiotics, enzymes, vitamins, hyaluronic acid, immune modulators, milk replacers, minerals, amino acids, carriers, in particular as mentioned above, coccidiostats, acid-based products and/or medicines, in particular antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

Immune modulators which may be used are for example antibodies, cytokines, spray-dried plasma, interleukins, or interferons, or combinations thereof.

The compositions of the invention may further comprise betaine and/or choline and/or other physiologically effective methyl group donors. The compositions may further comprise polyunsaturated fatty acids, in particular DHA and/or EPA.

Thus, a further embodiment of the invention is also a method of preparing a feed, food or pharmaceutical composition comprising mixing a compound or composition of the invention, in particular in an amount effective to enhance animal health, in particular gut health, with feed or food ingredients, such as proteins, lipids and/or carbohydrates, and optionally further beneficial substances, preferably as mentioned before, to provide a feed, food or pharmaceutical product. This method may comprise also a pelleting and/or extruding step. Thus, in a preferred embodiment of the invention the feed compositions are feed or food pellets and/or feed or food extrudates.

The food composition of the invention can in particular be a functional food, a food for special medical purposes, a nutraceutical or a dietary supplement. The dietary supplement can in particular be a pill, a tablet, a capsule or a liquid.

The compositions of the invention, in particular the food and feed compositions, may also be contained in a capsule, in particular in a bile-resistant capsule.

The compositions of the invention, in particular the food and feed compositions, may also comprise a coating, in particular an enteric coating, wherein the enteric coating preferably comprises one or more of the following substances: methyl acrylate-methacrylic acid copolymers, cellulose acetate phthalate (CAP), cellulose acetate succinate, Hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

The cosmetical composition of the invention is preferably a topical skin care composition.

According to the invention the term "skin" refers preferably to the skin itself, particularly to the human skin, but may also refer to the mucosa and skin adnexa, in so far that they include living cells, particularly hair follicles, hair roots, hair bulbs, the ventral epithelial layer of the nail bed (lectulus) as well as sebaceous glands and perspiratory glands.

Preferably according to the invention always an effective amount of the strains, fermentation broths and compositions of the invention is used in the embodiments of the invention. The term "effective amount" refers to an amount which effects at least one beneficial effect to the human skin, in particular with respect to the features as already mentioned before, in comparison to a human skin that has not been exposed to the microorganisms, microbial preparations or compositions of the invention, but may have been exposed to the same treatment and/or to the same cosmetical composition depleted by such microorganisms or microbial preparations.

The cosmetic or pharmaceutical composition according to the invention can be in any form suitable for application on the skin, e.g., a soap, a lotion, a serum, a spray, a jelly, a cream, a gel, a paste, a pomade, a balm, an ointment, a foam, a mousse, an emulsion, a stick, a suppository, a patch, a powder, a cleaning fluid or cleaning milk, a deodorant, an anti-perspirant, a salve, a hair conditioner or a shampoo. The composition can also be applied in a mask or in a band-aid, particularly in a gel reservoir mask or band-aid or matrix mask or band-aid.

The cosmetical compositions of the invention preferably have a pH in the range of between 3 to 8, in particular in the range of 4.0 to 8.0, more preferably in the range of 4.5 to 7.4, particularly preferably in the range of 5.0 to 7.2. The pH of the composition is preferably determined at 22°C after stirring for five minutes using a pH electrode calibrated in accordance with ISO 4319 (1977).

The application area can be the skin of any part of the body, particularly the facial skin, the scalp, the skin on hands and feet, the skin under the arms, as well as the mucous membrane, wherein the facial skin and the mucosa in the vaginal area are particularly preferred.

In a very preferred embodiment of the invention the cosmetical composition is a composition for topical application to the vaginal mucosa and is in particular selected from suppositories, creams and gels.

The cosmetical composition of the invention is preferably an emulsion, in particular a W/O or an O/W emulsion. This means that the composition contains preferably at least one surface-active substance as emulsifier or as dispersion agent. Emulsifiers act at the interphase to produce water or oil-stable adsorption layers that protect the dispersed droplets against coalescence and thereby stabilize the emulsion. Thus, emulsifiers, like surfactants, are composed of hydrophobic and hydrophilic molecular moieties. Hydrophilic emulsifiers preferably form O/W emulsions and hydrophobic emulsifiers preferably form W/O emulsions. An emulsion is understood to mean a dispersion of a liquid in the form of droplets in another liquid using an energy input to afford interphases stabilized with surfactants. The choice of this emulsifying surfactant or emulsifier depends on the materials being dispersed and the respective external phase as well as the fineness of the emulsion.

In addition to or instead of the emulsifiers as mentioned above the cosmetical compositions according to the invention may also comprise foaming, non-ionic, zwitterionic, anionic and/or cationic surfactants and/or biosurfactants as further surface-active ingredients.

The cosmetical or pharmaceutical skin care composition of the invention preferably comprises at least one further ingredient selected from phospholipids, sphingoid bases, free fatty acids, hyaluronic acid, protein hydrolyzates, polymers, light stabilizers, anti-perspirants, deodorants, peptides, amino acids, collagens, vitamins, extracts from plants and algae, cosmetic oils, emollients, antioxidants, preservatives, thickeners, viscosity regulators, stabilizers, hydrotropes, solids, fillers, film formers, conditioners, insect repellents, self-tanning agents, odor absorbers, solvents, perfumes and dyes.

A further subject of the invention is accordingly also the use of the microorganisms, microbial preparations and/or cosmetical compositions of the invention for treating the human skin.

A further subject of the invention is accordingly also a method of treating the human skin topically, wherein the microorganisms, microbial preparations and/or compositions of the invention are employed.

The cleaning composition of the invention is preferably a laundry composition.

The cleaning composition and in particular the laundry composition of the invention preferably contains at least one component, more preferably at least two or three components, selected from surfactants, enzymes, builders, solvents, preservatives, benefit agents, polymers, bleaching systems, antiredeposition aids, fibre protection agents, soil release agents, dye transfer inhibitors, fabric hueing agents, blueing dyes, enzyme stabilizing agents like boric acid, pH-regulators, emollients, emulsifiers, thickeners, viscosity regulators, stabilizers, UV photoprotective filters, antioxidants, hydrotropes, polyols, solids and fillers, film formers, pearlescent additives, deodorant and antiperspirant active ingredients, insect repellents, self-tanning agents, preservatives, conditioners, perfumes, dyes, odour absorbers, cosmetic active ingredients, care additives, superfatting agents, solvents and malodor removers.

A further subject of the invention is accordingly also the use of a microorganism or microbial preparation or of a composition of the invention for cleaning laundry.

A further subject of the invention is accordingly also a method of cleaning laundry, wherein a microorganism or microbial preparation or a composition of the invention, in particular a laundry composition, is employed.

The personal care articles of the invention preferably contain a water-absorbing gelling material, wherein the water-absorbing gelling material is preferably a water-absorbing composition comprising water-absorbing polymer structures, wherein the water-absorbing polymer structures are preferably based on partly neutralized, crosslinked polyacrylates. Very preferably, the personal care articles contain superabsorbent polymers.

Superabsorbent polymers (superabsorbers) are water-insoluble crosslinked polymers which are capable of absorbing and retaining under pressure large amounts of aqueous liquids, in particular body fluids, preferably urine or blood, with swelling and formation of hydrogels. In general, these uptakes of liquid are an amount of water at least 10 times or even at least 100 times the dry weight of the superabsorber or superabsorbent compositions. Due to these characteristic properties, these polymers are very suitable to be used in sanitary articles. A comprehensive overview of superabsorbers and superabsorbent compositions, their use and their preparation is given by F. L. Buchholz and A. T. Graham (editors) in "Modern Superabsorbent Polymer Technology", Wiley-VCH, New York, 1998.

Suitable water-absorbing gelling materials and methods of preparing them are described or mentioned in WO 2009/040106.

The microorganisms and microbial preparations can further be immobilized on the water-absorbing gelling material with aid of a binder. Suitable binders for this purpose are also disclosed in WO 2009/040106.

The personal care articles preferably contain besides at least one microorganism or microbial preparation of the invention at least one precursor of spermidine and/or agmatine, preferably L-arginine or a source of L-arginine, in particular as mentioned before.

A further subject of the invention is also the use of a microorganism of the species *Bacillus pumilus* or *Bacillus altitudinis* and/or of a microorganism according to the invention for producing spermidine or agmatine, wherein the microorganism is preferably selected from the strains *Bacillus subtilis* DSM 35086, *Bacillus subtilis* DSM 35087, *Bacillus subtilis* DSM 35085, *Bacillus subtilis* DSM 33561, *Bacillus pumilus* DSM 35088 *Bacillus pumilus* DSM 35089, *Bacillus pumilus* DSM 35090, *Bacillus pumilus* DSM 35091, *Bacillus pumilus* DSM 35093 and *Bacillus altitudinis* DSM 35092 and wherein production is preferably carried out in presence of a precursor of spermidine or agmatine, wherein the precursor of spermidine or agmatine is preferably selected from L-arginine, L-ornithine, L-methionine, putrescine or sources thereof and is preferably L-arginine or an arginine source, in particular dipeptides comprising L-arginine, proteins or protein hydrolysates, preferably prepared from wheat, wheat germs or soy, or an L-arginine derivative like L-arginine alpha-ketoglutarate or L-arginine ethylester.

A further subject of the invention is accordingly also a method for producing spermidine or agmatine, wherein a microorganism of the species *Bacillus pumilus* or *Bacillus altitudinis* and/or a microorganism according to the invention is cultivated in an appropriate medium, wherein the microorganism is preferably selected from the strains *Bacillus subtilis* DSM 35086, *Bacillus subtilis* DSM 35087, *Bacillus subtilis* DSM 35085, *Bacillus subtilis* DSM 33561, *Bacillus pumilus* DSM 35088 *Bacillus pumilus* DSM 35089, *Bacillus pumilus* DSM 35090, *Bacillus pumilus* DSM 35091, *Bacillus pumilus* DSM 35093 and *Bacillus altitudinis* DSM 35092 and wherein production is preferably carried out in presence of a precursor of spermidine or agmatine, wherein the precursor of spermidine or agmatine is preferably selected from L-arginine, L-ornithine, L-methionine, putrescine or sources thereof and is preferably L-arginine or an arginine source, in particular dipeptides comprising L-arginine, proteins or protein hydrolysates, preferably prepared from wheat, wheat germs or soy, or an L-arginine derivative like L-arginine alpha-ketoglutarate or L-arginine ethylester.

A further subject of the invention is also the use of a microorganism of the species *Bacillus pumilus* or *Bacillus altitudinis* and/or of a microorganism according to the invention in a process of extracting or enriching spermidine from food sources, in particular from wheat germs, soy, seeds or mushrooms.

A further subject of the invention is accordingly also a method of extracting or enriching spermidine from food sources, in particular from wheat germs, soy, seeds or mushrooms, wherein a microorganism of the species *Bacillus pumilus* or *Bacillus altitudinis* and/or a microorganism according to the invention is employed.

### Description of the figures

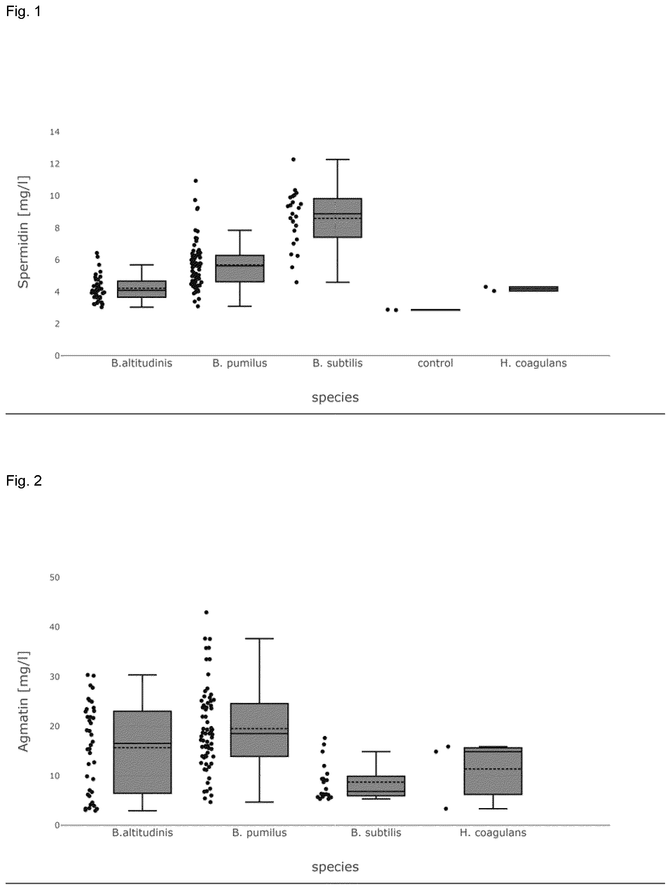

Figure 1 gives an overview about the amount of spermidine as produced by the tested *B. subtilis, B. pumilus* and B. *altitudinis* strains. As can be seen, all of the tested strains of the three species produced significant amounts of spermidine, so that it is reasonable to assume that the ability to produce spermidine surprisingly seems to be an intrinsic characteristic not only of the species B. *subtilis* - as already known before -, but also of the species B. *pumilus* and B. *altitudinis.*
Figure 2 gives an overview about the amount of agmatine as produced by the tested *B*. *subtilis, B. pumilus* and B. *altitudinis* strains. As can be seen, all of the tested strains of the three species produced significant amounts of spermidine, so that it is reasonable to assume that the ability to produce and accumulate spermidine is an intrinsic characteristic of all three such *Bacillus* species.

### Working Examples

### Example 1: Presence of genes involved in biosynthesis, transport, and degradation of spermidine

As previously known for spermidine producing strains, spermidine synthesis involves several key genes and pathways, with some known variation across species. Known to be embedded in the polyamine biosynthesis pathway of *Bacillus subtilis* are the enzymes *SpeABCDE*: *SpeA* (arginine decarboxylase; EC 4.1.1.19), *SpeB* (agmatinase; EC 3.5.3.11), *SpeC* (ornithine decarboxylase; EC 4.1.1.17], SpeD (S-adenosylmethionine decarboxylase; EC 4.1.1.50) and *SpeE* (spermidine synthase; EC 2.5.1.16). Of these enzymes, only *SpeE* is known to be conserved across the three domains of life, and catalyzes the last reaction from putrescine to spermidine.

These five enzymes are encoded by the *speABCDE* genes, which according to the invention were used in a genome mining approach to select putative spermidine-producing strains within a proprietary *Bacillus* biobank, generated by sequencing of own Bacillus strains of natural origin. Two bioinformatic search strategies were employed: (1) BLAST as the well-established search for protein homology [(Camacho, Coulouris et al. 2009)and (2) a more sensitive search with profile Hidden Markov Models (HMMs) to detect more sequence homologs(Potter, Luciani et al. 2018).

The proprietary biobank comprises several hundred sequenced isolates of, amongst others, *Bacillus altitudinis, Bacillus amyloliquefaciens, Bacillus pumilus, Bacillus safensis, Bacillus subtilis* and *Priestia megaterium* (formerly known as *Bacillus megaterium*). Reference genomes for the respective species as well as *for_Heyndrickxia coagulans* and *Bacillus toyonensis,* two known spermidine producers, were added with the latter serving as positive controls.

Beyond the key *speABCDE* genes, other genes related to spermidine synthesis and the pathway in *Bacillus* were included in the search: *metK* (S-adenosylmethionine synthetase; EC 2.5.1.6), *speF* (ornithine decarboxylase; EC 4.1.1.17], *paiAlspeG* (diamine N-acetyltransferase; EC:2.3.1.57), *adiA*/*pdaD* (arginine decarboxylase; EC 4.1.1.19), as well as known spermidine transport systems, such as: *potA* (spermidine/putrescine transport system ATP-binding protein; EC 7.6.2.11), *potB* (spermidine/putrescine transport system permease protein), *potC* (spermidine/putrescine transport system permease protein), and *potD* (spermidine/putrescine transport system substrate-binding protein).

In the proprietary biobank used, most *Bacillus* isolates selected as being putative spermidine producers encoded for *speABE* and *metK,* but surprisingly not for *potABCD.* This was in particular also true for the strains of the invention which turned out to be superior spermidine producers (see Table 1). Because these strains have the genetic potential to produce spermidine but lack the transporter system, it seems likely that another transporter, such as the *Bacillus subtilis* multidrug transporter *Blt* (Woolridge *et al.* 2017, PMID 9083003), is responsible for the efflux of spermidine. Basing on the results of the genomic screening, a selection of 57 genomically different and phylogenetically diverse *Bacillus* strains, hereof 26 *Bacillus pumilus,* 10 *Bacillus subtilis,* 21 *Bacillus altitudinis* strains, was identified as possible spermidine producers and was accordingly subjected to further screening (see Example 2).

**Table 1: Overview about identified putative genes related to spermidine production and export with respect to the best production strains**

| **Strain** | **Species** | **speA** | **speB** | **speE** | **metK** | **potA** | **potS** | **potC** | **potD** |
|---|---|---|---|---|---|---|---|---|---|
| DSM 33561 | *B. subtilis* | 2 | 1 | 1 | 1 | 0 | 0 | 0 | 0 |
| DSM 35085 | *B. subtilis* | 2 | 1 | 1 | 1 | 0 | 0 | 0 | 0 |
| DSM 35088 | *B. pumilus* | 2 | 1 | 1 | 1 | 1 | 0 | 0 | 0 |
| DSM 35089 | *B. pumilus* | 2 | 1 | 1 | 1 | 1 | 0 | 0 | 0 |
| DSM 35086 | *B. subtilis* | 2 | 1 | 1 | 1 | 0 | 0 | 0 | 0 |
| DSM 35090 | *B. pumilus* | 2 | 1 | 1 | 1 | 1 | 0 | 0 | 0 |
| DSM 35093 | *B. pumilus* | 2 | 1 | 1 | 1 | 1 | 0 | 0 | 0 |
| DSM 35091 | *B. pumilus* | 2 | 1 | 1 | 1 | 1 | 0 | 0 | 0 |
| DSM 35087 | *B. subtilis* | 2 | 1 | 1 | 1 | 0 | 0 | 0 | 0 |
| DSM 35092 | *B. altitudinis* | 2 | 1 | 1 | 1 | 1 | 0 | 0 | 0 |
| *Reference strain* | *B. toyocerin* | 2 | 1 | 2 | 1 | 1 | 1 | 1 | 1 |
| *H. coagulans* DSM1 | *H. coagulans* | 2 | 1 | 0 | 1 | 1 | 1 | 1 | 1 |

In Table 1 it can be seen that while previously known effective spermidine producers like *H. coagulans* DSM 1 and a known spermidine producer of the species *B. toyocerin* possess all transporter genes potABCE, the Bacillus strains which were identified as best spermidine producers according to the invention do not possess the genes potBCD, which arre deemed to be necessary for the formation of the spermidine exporter and thus for effective accumulation of spermidine in the fermentation broth.

### Example 2: Screening of spermidine- and agmatine-producing Bacillus pumilus, Bacillus altitudinis, and Bacillus subtilis

Based on the outcome of the genome mining described in Example 1, a selection of 57 genomically different *Bacillus* strains, hereof 26 *Bacillus pumilus,* 10 *Bacillus subtilis,* 21 *Bacillus altitudinis* strains, isolated from different environmental sources like soil and compost were screened regarding spermidine and agmatine formation in a complex nutrient-rich medium supplemented with spermidine precursors 07.5g/l L-methionine and 0.5g/l L-arginine at an initial pH of 7. *Heyndrickxia coagulans* (formerly *Bacillus coagulans*) DSM-1 was used as positive control for spermidine production.

For the screening, a single colony of each strain was precultured in a 100 ml shaking flask with10 ml Luria Bertani medium (Miller) containing 0.1% glucose ("LBG medium": 5 g/L yeast extract, 10g/L peptone from casein, 10 g/L sodium chloride (Merck, Germany), 1 g/L glucose monohydrate), supplemented with 0.5 g/L L-methionine ( Sigma-Aldrich, USA) and 0.5 g/l L-arginine (Merck, Germany) at 37°C, 200_rpm shaking frequency and aerobic conditions.

After 16 h a fresh culture of 2.5 ml LBG-medium was inoculated with an optical density of 0.1 at 660 nm for 24 h at 37°C with 300rpm shaking frequency under aerobic conditions. The strains were cultivated in 24 deep-well plates (Woulter-Duetz oxidish system, PreSens Precision Sensing GmbH, Germany) and the cultures were harvested by centrifugation (10 min, 4000 rpm) and the cell-free supernatant was subsequently analyzed analytically regarding the content of spermidine and agmatine.

The amount of produced spermidine and agmatine was compared to the control sample without bacteria inoculum. For the determination of spermidine and agmatine a liquid chromatographic separation via a LC-MS QToF system according to a respective standard substance of known concentration is used. Values presented in Figures 1 and 2 as well as in Table 2 show the determined concentrations of spermidine and agmatine.

### Quantification of Spermidine and Agmatine

### Sample Preparation (Derivatization):

Before final analysis a sample derivatization with benzoyl chloride is carried out (Schotten-Baumann-reaction). For this purpose, a 400µL aliquot of the sample material is mixed with 1ml of an aqueous 2M NaOH solution and 1mL methanol is added. Subsequently 10µL of benzoyl chloride (99%) is added to the mixture, vortexed and incubated at 30 °C for 20min. After addition of an aqueous saturated sodium chloride solution the derivatized sample material is extracted with 2 mL ether. A 100 µL aliquot of the organic layer is transferred into a vial, evaporated and residues re-dissolved in 160µL methanol. The methanolic solution is used for the analytical measurements.

All analytical measurements for the experiments have been carried out via a LC-MS QToF system. Applied LC unit belongs to 1290 Infinity II series connected to a mass spectrometer QToF 6546 (Agilent). No further dilution steps were carried out after the described derivatization procedure.

Peak identification was carried out via retention time and high-resolution molecular mass detection. Data evaluation was carried out via peak area and a quadratic calibration without zero. As an internal standard 1,6-diaminohexane was applied.

### Acquisition Method Details:

| | |
|---|---|
| Ion Source: | Dual AJS ESI (electrospray ionization) |

### Source Parameters

| | |
|---|---|
| Mass Range | m/z = 50-1000 |
| Scans per second | 6 |
| Fragmentation | 135 V |
| Polarity | Positive (Scan-Mode) |
| Gas temp. | 325 °C |
| Gas flow | 8 l/min |
| Nebulizer | 30 psig |
| Sheath Gas Temp | 350 °C |
| Sheath Gas Flow | 8 l/min |
| Capillary | 3000 V |

### Binary Pump

| | |
|---|---|
| Injection Volume | 1.00 µL |
| Flow | 0.50 mL/min |

### Solvent Composition

| | **Channel** | **ch. 1 Solv** | **Name 1** |
|---|---|---|---|
| **1** | A | 100.0% Water V.03 | +0.1%FA |
| **2** | B | 100.0% Methanol V.03 | |

### Gradient

| | **Time (min)** | **A (%)** | **B (%)** | **Flow (mL/min)** | **Pressure (bar)** |
|---|---|---|---|---|---|
| **1** | Start. Cond min | 40.00 % | 60 00 % | 0.500 mL/min | 600.00 bar |
| **2** | 5.00 min | 10.00 % | 90.00 % | 0.500 mL/min | 600.00 bar |
| **3** | 6.00 min | 10.00 % | 90.00 % | 0.500 mL/min | 600,00 bar |
| **4** | 6.10 min | 90.00 % | 10.00 % | 0.500 mUmin | 600.00 bar |
| **5** | 7.10 min | 90.00 % | 10.00 % | 0.500 mL/min | 600.00 bar |
| **6** | 7.20 min | 40.00 % | 60.00 % | 0.500 mL/min | 600.00 bar |

| | |
|---|---|
| Column Type: | Poroshell 120 EC-C18, 100 × 3 mm, 2,7µm, Agilent |
| Temperature: | 35.0 °C |

| | |
|---|---|
| Methodical error: | +/-15% |
| LOD (detection limit): | 0,1 mg/L |
| LOQ (quantification limit): | 0,3 mg/L |

As a result of the screening, the ability to produce and accumulate spermidine in the fermentation broth could surprisingly be identified as an intrinsic characteristic of the species *Bacillus pumilus* and *Bacillus altitudinis,* as depicted in Fig. 1.

Fig. 1 shows that all of the tested strains of those two species produced significant amounts of spermidine, so that it is reasonable to assume that the ability to produce spermidine is an intrinsic characteristic of these species. In addition *Bacillus subtilis* was confirmed to be a spermidine producing species.

Further, all of the tested *B. pumilus, B. altitudinis* and *B*. *subtilis* strains surprisingly produced and accumulated a significant amount of agmatine, a precursor of spermidine, in the fermentation broth, so that these strains could be identified not only as spermidine, but also as agmatine production strains.

Ten of the Bacillus strains as subjected to the screening were eventually identified to produce extraordinarily high levels of spermidine (up to 11mg/l) and/or agmatine (up to 40 mg/l), under the applied conditions (see Table 2). Of note, all of such strains produced higher concentrations of spermidine compared to *Heyndrickxia coagulans* DSM-1, which was used as reference strain and benchmark, and further also a higher amount of spermidine compared to other microbes reported to secrete spermidine before (*Heyndrickxia coagulans* YF1; (Suzuki, Fujiwara et al. 2023); *Bacillus subtilis* strains;(Lee, Kwon et al. 2023)).

**Table 2: Production of spermidine and agmatine by the best production strains**

| | | mean metabolite concentration in supernatants | |
|---|---|---|---|
| Strain | Species | Agmatine (mg/L) | Spermidine (mg/L) |
| DSM 35085 | *Bacillus subtilis* | 2.39 | 9.77 |
| DSM 35086 | *Bacillus subtilis* | 6.19 | 11.31 |
| DSM 35087 | *Bacillus subtilis* | 6.82 | 10.11 |
| DSM 33561 | *Bacillus subtilis* | 5.75 | 8.42 |
| DSM 35088 | *Bacillus pumilus* | 9.68 | 6.43 |
| DSM 35089 | *Bacillus pumilus* | 36.70 | 2.82 |
| DSM 35090 | *Bacillus pumilus* | 27.13 | 5.09 |
| DSM 35091 | *Bacillus pumilus* | 40.25 | 6.98 |
| DSM 35093 | *Bacillus pumilus* | 13.35 | 6.93 |
| DSM 35092 | *Bacillus altitudinis* | 29.22 | 6.31 |
| DSM 1 / ATCC 7050 | *Heyndrickxia coagulans* | 9.18 | 2.10 |

### References

Caffaratti, C., C. Plazy, V. Cunin, B. Toussaint and A. Le Gouellec (2022). "Bioengineering of Escherichia coli Nissle 1917 for Production and Excretion of Spermidine, a Key Metabolite in Human Health." Metabolites 12(11).
Camacho, C., G. Coulouris, V. Avagyan, N. Ma, J. Papadopoulos, K. Bealer and T. L. Madden (2009). "BLAST+: architecture and applications." BMC Bioinformatics 10: 421.
Chen, L., Y. Liu, G. Wu, N. Zhang, Q. Shen and R. Zhang (2017). "Beneficial Rhizobacterium Bacillus amyloliquefaciens SQR9 Induces Plant Salt Tolerance through Spermidine Production." Mol Plant Microbe Interact 30(5): 423-432.
Lee, K., S. H. Kwon, S. Song, D. Y. Lee, M. K. Park and Y. S. Kim (2023). "Comparative Analysis of Volatile and Non-Volatile Metabolites Derived from Bacillus subtilis Strains Producing Different Levels of Biogenic Amines." Metabolites 13(2).
Madeo, F., T. Eisenberg, F. Pietrocola and G. Kroemer (2018). "Spermidine in health and disease." Science 359(6374).
Potter, S. C., A. Luciani, S. R. Eddy, Y. Park, R. Lopez and R. D. Finn (2018). "HMMER web server: 2018 update." Nucleic Acids Research 46(W1): W200-W204.
Suzuki, H., Y. Fujiwara, K. Thongbhubate, M. Maeda and K. Kanaori (2023). "Spore-Forming Lactic Acid-Producing Bacterium Bacillus coagulans Synthesizes and Excretes Spermidine into the Extracellular Space." J Aqric Food Chem 71(25): 9868-9876.
Xie, S. S., H. J. Wu, H. Y. Zang, L. M. Wu, Q. Q. Zhu and X. W. Gao (2014). "Plant growth promotion by spermidine-producing Bacillus subtilis OKB105." Mol Plant Microbe Interact 27(7): 655-663.
Zou, D., Z. Zhao, L. Li, Y. Min, D. Zhang, A. Ji, C. Jiang, X. Wei and X. Wu (2022). "A comprehensive review of spermidine: Safety, health effects, absorption and metabolism, food materials evaluation, physical and chemical processing, and bioprocessing." Compr Rev Food Sci Food Saf 21(3): 2820-2842.

## Claims

1. Microorganisms and microbial preparations selected from the following group:
a) *Bacillus subtilis* DSM 35086;
b) *Bacillus subtilis* DSM 35087;
c) *Bacillus subtilis* DSM 35085;
d) *Bacillus subtilis* DSM 33561;
e) *Bacillus pumilus* DSM 35088;
f) *Bacillus pumilus* DSM 35089;
g) *Bacillus pumilus* DSM 35090;
h) *Bacillus pumilus* DSM 35091;
i) *Bacillus pumilus* DSM 35093;
j) *Bacillus altitudinis* DSM 35092;
k) A mutant of any of the strains according to (a) to (j), wherein the mutant has a sequence identity to the genomic DNA of said strain of at least 95 %, preferably of at least 97, 98 or 99 %, more preferably of at least 99.5, 99.8 or 99.9 %, in particular of at least 99.95, 99.98 or 99.99 %;
l) A preparation of any of the strains according to (a) to (k), wherein the preparation is preferably an optionally concentrated or dried fermentation broth, ferment, supernatant of a fermentation broth, cell-lysate or cell-extract of said strains, wherein the preparation may be a cell-free preparation or may contain living or dead cells or cell debris of such a microorganism or any combination thereof.

2. Microorganisms and microbial preparations according to claim 1, wherein the microorganisms are able to produce at least 2.5 mg/L, preferably at least 3, 4 or 5 mg/L, more preferably at least 5, 7 or 8 mg/L, above all at least 9 or 10 mg/L, spermidine in an appropriate fermentation medium, in particular in LBG medium supplemented with 0.5 g/L L-arginine and 0.5 g/L L-methionine, and/or wherein the mutants of the deposited strains are preferably able to produce at least 90 %, more preferably at least the same amount of spermidine in comparison to the parent strain, from which they are derived.

3. Microorganisms or microbial preparations according to claim 1 or 2, wherein the microorganisms are able to produce at least 2 mg/L, preferably at least 5 or 10 mg/L, more preferably at least 20 or 30 mg/L, above all at least 35 or 40 mg/L, agmatine in an appropriate fermentation medium, in particular in LBG medium supplemented with 0.5 g/L L-arginine and 0.5 g/L L-methionine, and/or wherein the mutants of the deposited strains are preferably able to produce at least 90 %, more preferably at least the same amount of agmatine in comparison to the parent strain, from which they are derived.

4. Composition containing at least one microorganism or microbial preparation according to any of claims 1 to 3, wherein the composition is preferably selected from feed, food, cosmetical, agricultural, cleaning and laundry compositions.

5. Composition according to claim 4, wherein the composition contains at least one, preferably at least two or three, further substances selected from carriers, further probiotics, prebiotics, enzymes, vitamins, minerals, amino acids, peptides, plant extracts and algal extracts.

6. Composition according to any of claims 4 or 5, wherein the composition is a composition for oral administration, preferably selected from food and feed compositions, food and feed supplements, food and feed additives, pet foods, dietary supplements, nutraceuticals and functional foods and preferably contains at least one further food or feed additive, in particular selected from proteins, carbohydrates, fats, feed concentrates, silage, mashfeed, immune modulators, milk replacers, acid-based products and/or medicines, in particular antibiotics.

7. Composition according to any of claims 4 or 5, wherein the composition is a cosmetical composition, preferably for topical application, and preferably contains at least one further additive selected from surfactants, emulsifiers, phospholipids, sphingoid bases, free fatty acids, hyaluronic acid, collagens, protein hydrolysates, polymers, light stabilizers, anti-perspirants, deodorants, cosmetic oils, emollients, antioxidants, preservatives, thickeners, viscosity regulators, stabilizers, hydrotropes, solids, fillers, film formers, conditioners, insect repellents, self-tanning agents, odor absorbers, solvents, perfumes and dyes.

8. Pharmaceutical composition containing at least one microorganism or microbial preparation according to any of claims 1 to 3 and a pharmaceutically acceptable carrier.

9. Composition according to any of claims 4 to 8, wherein the composition comprises at least one precursor of spermidine or agmatine, wherein the precursor of spermidine or agmatine is selected from L-arginine, L-ornithine, L-methionine, putrescine or sources thereof and is preferably L-arginine or an arginine source, in particular dipeptides comprising L-arginine, proteins or protein hydrolysates, preferably prepared from wheat, wheat germs or soy, or an L-arginine derivative like L-arginine alpha-ketoglutarate or L-arginine ethylester.

10. Composition according to any of claims 4 to 9, wherein the composition comprises living cells of at least one microorganism according to any of claims 1 to 3, preferably in an amount of from 1×10² to 1×10¹² CFU, more preferably in an amount of 1×10³ to 1×10¹⁰ CFU, above all in an amount of 1×10⁴ to 1×10⁹ CFU, wherein the microorganisms are preferably present exclusively or mainly as endospores.

11. Composition according to any of claims 4 to 10, wherein the composition is a coated composition and/or contained in a capsule, wherein the coated composition comprises preferably an enteric coating, in particular comprising methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate or zein or combinations thereof and wherein the capsule is preferably a bile-resistant capsule.

12. Composition according to any of claims 4 to 11 for use in the treatment, prevention or mitigation of a disease selected from dementia, Alzheimer's disease, Parkinson's disease, arthritis, hypertension, arterial stiffness, heart failure, myopathy, sepsis or cardiovascular diseases and/or for use in increasing the life span, promoting longevity, improving male or female fertility, promoting hair or nail growth, improving immunogenicity after vaccination, improving mitochondrial respiration.

13. Product comprising at least one microorganism or microbial preparation according to any of claims 1 to 2, wherein the product is preferably a dietary supplement, agricultural product or a personal care article, wherein the personal care article is preferably a disposable absorbent article, in particular selected from sanitary napkins, panty liners, tampons, diapers, incontinent pads, breast pads, perspiration pads, human or animal waste management devices and interlabial pads.

14. Non-medical use of a microorganism or microbial preparation according to any of claims 1 to 3 or of a composition according to any of claims 3 to 11 for improving skin firmness, elasticity, viscoelasticity, resilience or hydration, for reducing or preventing wrinkles or skin irritation or for promoting hair and nail growth.

15. Use of a microorganism of the species *Bacillus pumilus* or *Bacillus altitudinis* and/or of a microorganism according to any of claims 1 to 3 for producing spermidine or agmatine, wherein the microorganism is preferably selected from the strains *Bacillus subtilis* DSM 35086, *Bacillus subtilis* DSM 35087, *Bacillus subtilis* DSM 35085, *Bacillus subtilis* DSM 33561, *Bacillus pumilus* DSM 35088 *Bacillus pumilus* DSM 35089, *Bacillus pumilus* DSM 35090, *Bacillus pumilus* DSM 35091, *Bacillus pumilus* DSM 35093 and *Bacillus altitudinis* DSM 35092 and wherein production is preferably carried out in presence of a precursor of spermidine or agmatine, wherein the precursor of spermidine or agmatine is preferably selected from L-arginine, L-ornithine, L-methionine, putrescine or sources thereof and is preferably L-arginine or an arginine source, in particular dipeptides comprising L-arginine, proteins or protein hydrolysates, preferably prepared from wheat, wheat germs or soy, or an L-arginine derivative like L-arginine alpha-ketoglutarate or L-arginine ethylester.

16. Use of a microorganism of the species *Bacillus pumilus* or *Bacillus altitudinis* and/or of a microorganism according to any of claims 1 to 3 in a process of extracting or enriching spermidine from food sources, in particular from wheat germs, soy, seeds or mushrooms.
